# NEUE EUROPÄISCHE PATENTSCHRIFT

(11) **EP 1 445 387 B2**
(45) Veröffentlichungstag und Bekanntmachung des Hinweises auf die Entscheidung über den Einspruch: **28.10.2009**
(45) Hinweis auf die Patenterteilung: 11.04.2007
(21) Anmeldenummer: 04002036.4
(22) Anmeldetag: 30.01.2004
(51) Int. Cl.: F24F 3/16, E03D 9/052, E03D 9/05

(54) **Deodorisierungsvorrichtung und -verfahren für Toiletten**
Deodorizing apparatus and method for toilets
Dispositif et procédé de désodorisation pour toilettes

(30) Priorität: 03.02.2003 DE 20301640 U
(43) Veröffentlichungstag der Anmeldung: 11.08.2004
(60) Teilanmeldung: 06113855.8
(73) Patentinhaber: VILLEROY & BOCH AG, 66688 Mettlach (DE)
(72) Erfinder: Czapla, Christian, Dr., 66793 Saarwellingen (DE); Becker, Ralf, 66663 Merzing (DE)
(74) Vertreter: Gritschneder, Martin

(56) Entgegenhaltungen:
- EP-A- 0 331 192
- EP-A- 0 331 192
- WO-A-01/62306
- WO-A-2004/014442
- DE-A- 4 334 956
- DE-A- 10 007 523
- DE-A- 10 111 445
- DE-A- 19 651 402
- DE-A- 19 810 497

## Beschreibung

Die Erfindung betrifft eine Vorrichtung und ein Verfahren zum Vermeiden von lästigen Gerüchen auf Toiletten, wobei mit einem Lüfter Luft aus dem Innenbereich des WC-Beckens abgesaugt wird und mittels einer Ionisationseinrichtung Luftionen erzeugt werden, die mit der abgesaugten Luft in Kontakt gebracht werden, wodurch die Geruchsmoleküle oxidiert und damit zerstört werden.

Aus US-A-2 001 592 ist eine Vorrichtung bekannt, bei der aus dem Inneren des WC-Beckens die Luft mittels des Lüfters abgesaugt wird und durch die Ionisationseinrichtung geleitet wird, um Geruchsmoleküle zu oxidieren. Der Lüftermotor und die Ionisationseinrichtung werden durch einen Schalter eingeschaltet, der durch das Herunterklappen des WC-Sitzbrettes betätigt wird. Der Lüfter und die Ionisationseinrichtung sind in einem Gehäuse untergebracht, das seitlich neben der Schwenkachse des WC-Sitzbrettes angebracht ist.

Aus US-A-6 163 893 ist es bei einer weiteren solchen Vorrichtung bekannt, die Luft über mehrere Öffnungen im WC-Sitzbrett anzusaugen und dann durch die Ozon erzeugende Einrichtung und ein Aktivkohlefilter zu schicken.

Aus JP-A-05168687 ist es bei einer solchen Vorrichtung bekannt, die Luft noch durch einen Katalysator zu leiten. Die Betriebsstunden des Katalysators werden aufsummiert, und die Arbeitsweise der Ionisationseinrichtung wird der abnehmenden Leistungsfähigkeit des Katalysators angepasst.

Aus EP-B1-0 331 192, Fig. 12, ist eine ähnliche Anordnung bekannt, wobei die Luft durch das Überlaufrohr des Spülkastens abgesaugt wird und aus dem Spülkasten durch die Ionisiereinrichtung und einen Katalysator gesaugt wird. Die Steuerung der Anlage erfolgt über einen Licht-Detektor am Sitzbrett.

Aus JP-A-03290538 ist eine solche Toiletten-Deodorierungsvorrichtung bekannt, bei der die Benutzung der Toilette mittels eines Sensors erkannt wird und daraufhin die Vorrichtung eingeschaltet wird. Nach Beendigung der Benutzung bleibt sie noch eine vorgegebene Zeitspanne eingeschaltet. Ähnliche Vorrichtungen sind auch aus JP-A-2001262663, JP-A-09195359, JP-A-07279213 und JP-A-07268928 bekannt.

Aus EP-A-0 567 775 ist es bekannt, Raumluft für Wohn- und Arbeitsräume mittels eines Klimazentralgerätes aufzubereiten. Vor der Behandlung wird die Raumluft mit einem Schadstoff-Sensor auf oxidierbare Schadstoffe analysiert. Die Schadstoffe werden mittels Ozon aus einem Oxidator oxidiert, wobei die Menge des im Oxidator erzeugten Ozons in Abhängigkeit von dem Signal des Schadstoff-Sensors gesteuert wird. Die Raumluft wird ferner in einer Filtereinrichtung behandelt, wobei noch vorhandenes Ozon in stabilen, molekularen Sauerstoff umgewandelt wird. Das in dem Ozonisator erzeugte Ozon wird zusätzlich von einem Ozon-Sensor gesteuert und geregelt, der nach der Filtereinrichtung angeordnet ist.

Aus EP-A-0 331 162 ist ein Verfahren zum Deodorisieren eines Toilettenraums bekannt, bei dem durch einen Schalter am Sitzbrett eine Vorrichtung eingeschaltet wird, die die Luft aus dem WC-Becken absaugt und mittels Ozon die vorhandenen Geruchsmoleküle zerstört. Die Luft wird ferner durch ein Katalysatorbett und ein Aktivkohlefilter geleitet. Mittels eines danach angeordneten Ozonkonzentrationssensors wird überschüssiges Ozon aufgespürt und in Abhängigkeit davon wird die Ionisationsvorrichtung gesteuert.

Aus DE-A-101 11 445 ist ein Luftreinigungsgerät bekannt, bei dem die Luft an einer Ionisationsröhre und einem Gassensor vorbei geführt wird. Der Gassensor spürt Geruchsmoleküle auf und erzeugt daraus ein Signal zur Steuerung der Ionisationsröhre. Die Drehzahl des Lüfters kann zusätzlich derart geregelt werden, dass die von dem Gassensor gemessene Luftqualität einem vorgegebenen Sollwert entspricht. Mit dieser zusätzlichen Stellgröße wird die Einstellung der produzierten Ionen und/oder Radikalen und/oder Ozon durchgeführt. Anspruch 1 geht von dieser Druckschrift als nächstem Stand der Technik aus.

Die Erfindung, wie sie in den Ansprüchen 1 und 6 angegeben ist, hat die Aufgabe, eine Vorrichtung und ein Verfahren zum Vermeiden lästiger Gerüche auf Toiletten zu schaffen, mittels denen einerseits Gerüche sehr wirksam und zuverlässig unterdrückt werden können und anderseits die Produktion von überschüssigem Ozon vermieden wird.

Durch die Steuerung der Ionisierungseinrichtung in Abhängigkeit von der Konzentration der Geruchsmoleküle wird erreicht, dass die von der Ionisationseinrichtung produzierte Menge an Luftionen und Ozon gerade etwa so groß ist, wie es zur Oxidation der Geruchsmoleküle notwendig ist. Die Erzeugung einer wesentlichen Menge überschüssiger Luftionen und überschüssigen Ozons und der damit verbundene beißende Geruch werden vermieden.

Der Lüfter wird mittels des Sensorsignals in der Weise gesteuert, dass der Lüfter mit einer ersten, relativ niedrigen Leistung arbeitet, wenn das Steuersignal keine oxidierbaren Geruchsmoleküle anzeigt, und dass er mit einer zweiten, höheren Leistung arbeitet, wenn das Steuersignal das Vorhandensein oxidierbarer Geruchsmoleküle in der abgesaugten Luft anzeigt.

Der Lüfter arbeitet im Bereitschaftszustand, d.h., wenn der Sensor keine Geruchsmoleküle feststellt, etwa mit halber Leistung, während er dann, wenn der Sensor das Vorhandensein von Geruchsmolekülen feststellt, auf volle Leistung umgeschaltet wird. Gleichzeitig mit dem Hochfahren des Lüfters wird die Ionisierungseinrichtung aktiviert. Die Deodorisierungsvorrichtung fängt dadurch praktisch verzögerungsfrei zu arbeiten an. Ein weiterer Vorteil besteht darin, dass die Deodorisierungsvorrichtung nur aktiviert wird, wenn es tatsächlich notwendig ist.

Dadurch, dass der Sensor bei dieser Ausgestaltung der Erfindung ständig aktiv ist und der Lüfter im Bereitschaftszustand kontinuierlich mit niedriger Leistung läuft, spricht eine solche Deodorisierungsvorrichtung sehr rasch und automatisch an, wenn die Toilette benutzt wird. Zusätzliche Erkennungseinrichtungen oder Schalter am WC-Sitzbrett sind nicht notwendig.

Um die Benutzung der Toilette festzustellen und die Vorrichtung zu aktivieren, kann unter dem Sitzbrett jedoch ein Schalter angeordnet sein, durch den beim Niederdrücken des Sitzbrettes ein Stromkreis geschlossen wird. Es können aber auch bekannte Einrichtungen wie kapazitive Sensoren oder Infrarotsensoren verwendet werden, um die Gegenwart einer Person und damit die Benutzung der Toilette festzustellen.

Elektronische Steuerungen müssen von Zeit zu Zeit rückgesetzt werden, um Fehlfunktionen zu vermeiden. Ein solcher Reset findet zwangsläufig beim Einschalten des Gerätes statt. Da bei dieser Ausgestaltung der Erfindung das Deodorisierungsmodul ständig eingeschaltet ist, ist die Steuerung so ausgelegt, dass in bestimmten Zeitabständen, z.B. alle 24 Stunden, ein Reset durchgeführt wird.

Der Sensor kann in Richtung der von dem Lüfter erzeugten Luftströmung vor, hinter oder neben der Ionisierungseinrichtung angeordnet sein. Wichtig ist nur, dass der Sensor innerhalb des von dem Lüfter erzeugten Luftstroms angeordnet ist, bevor dieser Luftstrom wieder in den Raum austritt. Wenn der Sensor hinter der Ionisierungseinrichtung angeordnet ist, so ist bei der Festlegung der Steuerungskurve zu berücksichtigen, dass ein Teil der oxidierbaren Geruchsmoleküle bereits aufoxidiert ist, bevor sie den Sensor erreichen konnten. Die Abhängigkeit der Leistung der Ionisierungseinrichtung von dem Sensorsignal hat dann zumindest zum Teil den Charakter einer Regelung.

Der Sensor hat eine dreifache Funktion: Erstens wird die Ionisierungseinrichtung eingeschaltet, sobald der Sensor oxidierbare Geruchsmoleküle feststellt, zweitens steuert der Sensor in Abhängigkeit von der Konzentration der festgestellten Geruchsmoleküle die Leistung der Ionisierungseinrichtung und drittens wird die Leistung des Lüfters erhöht, sobald der Sensor oxidierbare Geruchsmoleküle feststellt.

Der Sensor ist vorzugsweise ein Halbleiter-Zinnoxid-Sensor, der oxidierbare Gasbestandteile in der Luft feststellt. Derartige Sensoren sind allgemein bekannt und zum Beispiel von der Firma Fis Inc., 2-5-26, Hachizuka, Ikeda, Osaka, 563-0024 Japan, erhältlich. In einer besonders bevorzugten Ausführungsform der Erfindung wird ein H₂-Sensor verwendet. Wasserstoff ist zwar ein geruchloses Gas, kann jedoch als Leitgas verwendet werden, da es immer bei der menschlichen Verdauung gebildet wird, während die anderen Gasbestandteile z. B. Methan oder H₂S in Abhängigkeit von der aufgenommenen Nahrung im größeren oder kleineren Umfang oder überhaupt nicht gebildet werden. Gleichzeitig hat Wasserstoff den Vorteil, dass es nicht in Parfums und ähnlichen Kosmetika verwendet wird, so dass ein fälschliches Ansprechen des Sensors auf derartige Kosmetika verhindert wird. Ein H₂-Sensor hat damit den Vorteil, dass er einerseits sicher anspricht, wenn irgendwelche Geruchsmoleküle oder Luftbestandteile vorhanden sind, die von der menschlischen Verdauung herrühren, und andererseits ein fälschliches Ansprechen auf z. B. Kosmetika vermieden wird. Geeignete H₂-Sensoren sind ebenfalls von der Firma Fis Inc. erhältlich (siehe Products Review, Sensors and Systems Technology, revised June, 1998, Version 4.2 der Firma Vis Inc.), z. B. das Modell SB 19.

Die Deodorisierungsvorrichtung ist zweckmäßig als ein kompaktes Modul ausgebildet, das den Lüfter, den Sensor, die Ionisierungseinrichtung mit Ionisierungsröhre und Hochspannungseinheit, die Steuerung und die Stromversorgung für diese Komponenten enthält. Die Stromversorgung erfolgt dabei zweckmäßig über das Stromnetz mittels eines Netzteils mit einem Netztrafo. Der Lüfter saugt über ein Ansaugrohr Luft an und fördert sie zu dem Sensor und der Ionisierungseinrichtung.

Die Ionisierungsröhre kann ein Glas-Hohlzylinder mit einem Außendurchmesser von 20 mm, einer Zylinderhöhe von 50 mm und einer Wandstärke von 0,8 mm sein. Auf der Innen- und Außenseite des Glaszylinders sind flächige, gitterförmige Elektroden angebracht. Die Enden der zylinderförmigen Röhre können offen oder verschlossen sein.

Die Hochspannungseinheit der Ionisierungseinrichtung erzeugt eine Spannung von 1 bis 1,8 kV mit einer Frequenz von 8 bis 13 kHz, bei der vorausgehend beschriebenen Ionisierungsröhre vorzugsweise eine Wechselspannung von 1,5 kV bei einer Frequenz von 10 kHz. Die Wechselspannung wird in Form von Rechteckimpulsen mit einer Frequenz z.B. im Bereich von 50 Hz an die Elektroden der Ionisationsröhre angelegt. Die Steuerung der Ionisationsleistung, d.h. der Menge der erzeugten Luftionen und der Ozonmoleküle, wird dadurch gesteuert, dass die Dauer der Rechteckimpulse und deren Abstand verändert wird (Tastverhältnis). An der Ionisationsröhre liegt damit entweder keine Spannung an oder eine bestimmte fest eingestellte Hochspannung, z.B. 1,5 kV. Wenn Ionisation stattfindet, dann erfolgt sie dadurch mit der einmal gefundenen optimalen Spannung, bei der die Menge der erzeugten Luftionen und der Ozonmoleküle pro Zeiteinheit weitgehend konstant ist.

Der Lüfter kann einen Axiallüfter mit einer maximalen Leistung von etwa 9 bis 12 Liter pro Minute sein. Über ein Ansaugrohr wird die Luft aus dem Inneren des WC-Beckens angesaugt und zu dem Deodorisierungsmodul geleitet. Das Ansaugrohr kann ein eigenes Rohr sein. Mit geeigneten Adaptern kann auch das Spülrohr oder das Überlaufrohr als Ansaugrohr fungieren. Die behandelte Luft wird über eine Auslassöffnung in dem Gehäuse des Deodorisierungsmoduls ausgeblasen, wobei diese Öffnung im Allgemeinen der Öffnung diametral gegenüberliegt, an der das Ansaugrohr in das Gehäuse mündet. Die behandelte Luft kann auch über ein Auslassrohr in den Raum abgeleitet werden. Dadurch wird die Zeitspanne verlängert, die zur Verfügung steht, um die oxidierbaren Geruchsmoleküle aufzuoxidieren. Die Geruchsmoleküle und die Luftionen und Ozonmoleküle sind während der Durchströmung des Auslassrohrs noch räumlich eingeschlossen, so dass sie miteinander reagieren können. Als ein solcher Reaktionsraum kann auch der Hohlraum auf der Rückseite eines WC-Beckens oder der Raum innerhalb eines Spülkastens dienen.

Die Deodorisierungsvorrichtung kann Teil einer Toilettenausstattung sein, die ein übliches WC-Becken und eine Spüleinrichtung mit einem Spülrohr aufweist. Bei der Spüleinrichtung kann es sich um einen Spülkasten handeln, der über das Spülrohr mit dem WC-Becken verbunden ist.

Das Deodorisierungsmodul (DOM) kann im hinteren Bereich des WC-Beckens angeordnet werden, bspw. unterhalb des Abflussrohrs oder Geruchsverschlusses, wo im allgemeinen ausreichend Platz zur Verfügung steht. Angesaugt wird die Luft aus dem Inneren des WC-Beckens über einen innerhalb des WC-Sitzes verlaufenden Kanal, der über eine Gelenkverbindung im Scharnier des WC-Sitzes mit dem Ansaugrohr des Deodorisierungsmoduls verbunden ist. In der Keramik des WC-Beckens kann eine zusätzliche, nach unten führende Öffnung im Bereich der Scharnierbefestigung vorgesehen sein. Statt über einen Kanal im WC-Sitz die Luft abzusaugen, besteht auch die Möglichkeit, eine eigene Öffnung neben dem Spülrohrauslass im oberen Bereich des WC-Beckens vorzusehen, über die die Luft abgesaugt und zum Deodorisierungsmodul geführt wird. An einem eventuell vorhandenen Spülkasten und an den Anschlüssen des Spülkastens und des Abflusses sind dann keine Veränderungen notwendig.

Das Deodorisierungsmodul kann auch im unteren Bereich des Spülkastens oder im hinteren Bereich des WC-Beckens neben oder unter dem WC-Abflussstutzen angeordnet werden, wobei die Luft aus dem WC-Becken über den Spülrohrauslass abgesaugt und über eine Abzweigung aus dem Spülrohr zum Deodorisierungsmodul geleitet wird.

Das Ansaugrohr kann auch vom Rand des WC-Beckens innerhalb des waagerechten Teils des Spülrohrs zurück verlaufen, dem Spülrohr ein kurzes Stück innerhalb des senkrechten Teils folgen, so dass kein Wasser über das Ansaugrohr zum Deodorisierungsmodul gelangen kann, und dann seitlich aus dem Spülrohr austreten und zum Deodorisierungsmodul führen.

Das Deodorisierungsmodul kann sich im Spülkasten über dessen Flutungslinie befinden. Als Ansaugrohr fungieren das Spülrohr und das Überlaufrohr, das unterhalb des Spülventils von dem Spülrohr abzweigt. Mittels eines Adapters wird das obere Ende des Überlaufrohrs in einem U-förmigen Kanal nach unten umgeleitet. Das Deodorisierungsmodul wird auf den Adapter aufgesetzt, wobei die Ansaugöffnung des Deodorisierungsmoduls in den U-förmigen Kanal mündet. Bei gefülltem Spülkasten wird Luft über das Überlaufrohr und das Spülrohr nur aus dem Inneren des WC-Beckens angesaugt, da das nach unten zeigende Ende des U-förmigen Kanals von dem im Spülkasten stehenden Wasser verschlossen ist.

Das Ansaugrohr kann in dem Überlaufrohr und dem Spülrohr des Spülkastens nach unten geführt werden, wobei sich die Einlassöffnung des Ansaugrohrs im Spülkasten über dessen Flutungslinie befindet. Das Ansaugrohr tritt zwischen Spülkasten und WC-Becken aus dem Spülrohr aus und verläuft zum Deodorisierungsmodul, das sich in dem Hohlraum auf der Rückseite des WC-Beckens befindet.

Das erfindungsgemäße Deodorisierungsmodul ist jeweils dasselbe. Es ist sowohl für WC-Becken mit Wandmontage als auch für Kombi-WCs geeignet. Bei der Wandmontage wird es entweder mittels einer Manschette vom Abflussrohr abgehängt, wobei das Ansaugrohr dann an der Oberseite des aus der Wand herausgeführten Teils des Spülrohrs angesetzt ist und um das Abflussrohr herum zu dem Deodorisierungsmodul verläuft. Mittels elastischer Klemmelemente kann es auch in den Hohlraum auf der Rückseite des WC-Beckens eingesetzt werden. Die Klemmelemente können einfache Kunststoffstücke sein, die in ihrer Stärke an die Abmessungen des Hohlraums angepasst werden. Sie können auch seitlich von dem Deodorisierungsmodul abstehende Schrauben sein, deren Kopf mit einer Kunststoffauflage gegen die Innenseite des Hohlraums drückt. Durch Eindrehen bzw. Herausdrehen können die Schrauben an die Abmessungen des Hohlraums angepasst werden.

Bei einigen der erwähnten Einbaumöglichkeiten des Deodosierungsmoduls verläuft das Ansaugrohr innerhalb des Spülrohrs oder Überlaufrohrs. Deren Querschnitt muss ggf. vergrößert werden, damit das Spülwasser genügend schnell in das WC-Becken einläuft. Die lichte Weite des Ansaugrohrs wird jeweils in Abhängigkeit von seiner Länge so gewählt, dass die gewünschte Ansaugleistung von etwa 9 bis 12 Liter pro Minute erreicht wird.

Es kann sich bisweilen das Problem ergeben, dass die Luft nach dem Durchgang durch die Deodorisierungsvorrichtung noch einen geringen Anteil an Ozon enthält. In diesen Fällen kann es vorteilhaft sein, der Deodorisierungsvorrichtung am Auslassende noch ein Aktivkohlefilter nachzuschalten. Da der größte Anteil der oxidierbaren Gase und des Ozons bereits vor dem Aktivkolifilter miteinander reagieren, wird das Aktivkohlefilter wenig belastet, so dass es eine sehr hohe Standzeit hat. In bekannter Weise kann das Aktivkohlefilter z.B. durch Erhitzen regeneriert werden.

Ein besonderer Vorteil der erfindungsgemäßen Deodorisierungsvorrichtung besteht darin, dass sie in bestehende WCs eingebaut werden kann, ohne die Keramik des WC-Beckens zu verändern. Der Stromanschluss kann sowohl aufputz als auch unterputz erfolgen.

Ein weiterer Vorteil der erfindungsgemäßen Deodorisierungsvorrichtung besteht darin, dass nicht nur lästige Gerüche auf der Toilette vermieden werden, sondern auch eine Entkeimung der Luft stattfindet.

Ausführungsbeispiele der Erfindung werden nachfolgend anhand der Zeichnungen näher erläutert. Es zeigen:
- Fig. 1: in einer schematischen Darstellung das Deodorisierungsmodul;
- Fig. 2: die Anordnung der einzelnen Bauelemente des Deodorisierungsmoduls auf einer Platine;
- Fig. 3: bis 13 mehrere Anwendungsbeispiele der erfindungsgemäßen Deodorisierungsvorrichtung.

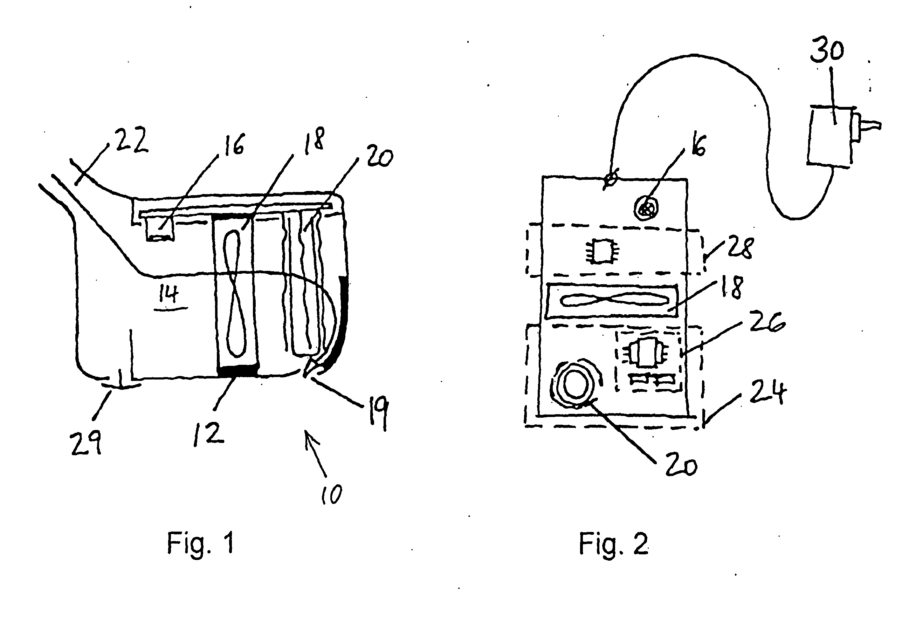

Wie in Fig. 1 gezeigt, ist das Deodorisierungsmodul (DOM) 10 in einem kompakten Gehäuse 12 untergebracht, durch das ein Strömungskanal 14 verläuft, in dem in Strömungsrichtung hintereinander ein Sensor 16, ein Lüfter 18 und eine Ionisationsröhre 20 angeordnet sind. Der Lüfter 18 kann auch an jeder anderen Stelle innerhalb des Strömungskanals 14 angeordnet sein. Er saugt Luft über ein Ansaugrohr 22 an, treibt sie durch den Strömungskanal 14 und bläst sie nach der Behandlung über eine Auslassöffnung 19 wieder in die Umgebung aus. Die Ionisationsröhre 20 ist Teil einer Ionisationseinrichtung 24 (Fig. 2), die noch eine Hochspannungseinheit 26 aufweist. Eine Steuereinheit 28 verarbeitet die Signale des Sensors 16 und steuert die Ionisationseinrichtung 24 in Abhängigkeit von den Sensorsignalen. Die Stromversorgung aller Komponenten erfolgt über ein Netzteil 30 in einem Netzstecker mit einer Ausgangsspannung von 12 Volt. Die Reihenfolge von Sensor 16, Lüfter 18 und Ionisationsröhre 20 ist dabei beliebig. Der Sensor 16, die Steuereinheit 28, der Lüfter 18, die Ionisationsröhre 20 und die Hochspannungseinheit 26 sind alle auf der gleichen Seite einer gemeinsamen Platine 32 montiert. Die Platine 32 ist oben in dem Gehäuse 12 befestigt, wobei die Montageseite mit den einzelnen Komponenten nach unten zeigt. Der Lüfter 18 füllt dabei den gesamten Querschnitt des Gehäuses 12 aus und auch die Ionisationsröhre 20 erstreckt sich bis nahe zur Unterseite des Gehäuses 12 (Fig. 1). Das Ansaugrohr 22 mündet auf der in Fig. 1 linken oberen Kante in das Gehäuse 12 und die Auslassöffnung 19 befindet sich an der rechten unteren Kante. Das DOM 10 hat an der Unterseite ein Notventil 29, über das eventuell eintretendes Wasser abgelassen wird. Das Notventil 29 ist als Pilzventil ausgeführt und öffnet bereits bei geringem Wasserdruck. Wie nachfolgend noch erläutert wird, sind die Mittel zum Ansaugen der Luft aus dem Beckeninneren jedoch so ausgelegt, dass kein Wasser in das DOM 10 eintreten kann.

Der Sensor 16 ist typischerweise ein Halbleiter-Zinnoxid-Sensor, der oxidierbare Gasbestandteile in der Luft feststellt. Der elektrische Widerstand des Sensors 16 ändert sich in Abhängigkeit von der Konzentration der oxidierbaren Luftbestandteile, die sich in der Luft befinden, die an den frei liegenden Oberflächen des Sensors 16 vorbei strömt.

Die Ionisationsröhre 20 besteht aus einem Glas- oder Keramikzylinder mit zwei Elektroden auf der Innenseite und der Außenseite. Die Hochspannungseinheit 26 erzeugt eine elektrische Wechselspannung von 1,5 kV und 10 kHz und diese Wechselspannung wird an die beiden Elektroden angelegt, wodurch Luftionen und aktiver Sauerstoff nach dem Prinzip der stillen oder dielektrisch behinderten Entladung erzeugt werden. Die Ionisationseinrichtung arbeitet im Impulsbetrieb, d.h. die Hochspannungseinheit 26 erzeugt eine die Hochspannung in Form einer Folge von Rechteck-Hochspannungsimpulsen. Dadurch wird erreicht, dass die Ionisationseinrichtung 24 immer mit der optimalen Spannung betrieben wird, bei der stabil und reproduzierbar überwiegend Luftionen und nur ein geringer Anteil an aktiven Sauerstoffatomen erzeugt werden.

Die Steuereinheit 28 steuert das DOM 10 in der Weise, dass im Ruhezustand, d.h., wenn der Sensor 16 kein Signal abgibt, das auf das Vorhandensein von oxidierbaren Luftbestandteilen hinweist, der Lüfter 18 etwa mit seiner halben Nennleistung läuft und die Ionisationseinrichtung 24 stromlos ist. Sobald der Sensor 16 meldet, dass oxidierbare Luftbestandteile in der angesaugten Luft vorhanden sind, wird der Lüfter 18 auf volle Leistung geschaltet und wird die Ionisationseinrichtung 24 eingeschaltet, wobei die Leistung der Ionisationseinrichtung 24 in Abhängigkeit von der Menge der oxidierbaren Luftbestandteile gesteuert wird. Die Steuereinheit 28 steuert die Leistung der Ionisationseinrichtung 24 durch Verändern der Impulsdauer und/oder der Impulsabstände, wobei die Maximal-Spannung jedes Impulses im Wesentlichen konstant bei etwa 1,5 kV liegt. Die Steuerung der Ionisationsleistung erfolgt in Abhängigkeit von dem Widerstandswert des Sensors 16 mit dem Ziel, gerade die Menge an Luftionen und Sauerstoffatome zu erzeugen, die notwendig ist, um die oxidierbaren Luftbestandteile zu oxidieren und damit zu zerstören. Die Sauerstoffatome führen zur Bildung von Ozon. Überschüssige Sauerstoffatome würden als beißender Ozongeruch wahrgenommen.

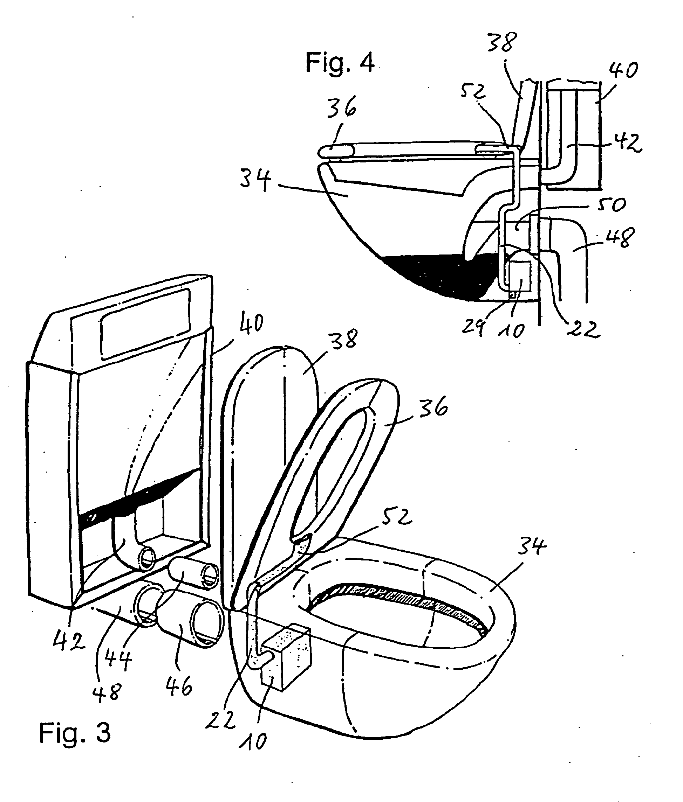

Die Figuren 3 bis 15 zeigen jeweils eine Toilettenausstattung mit einem WC-Becken 34, an dem ein Sitzbrett 36 und ein Deckel 38 in üblicher Weise mittels eines Scharniers befestigt sind. Die Toilette weist ferner einen Spülkasten 40 mit einem Spülrohr 42 auf, das in mehreren Auslassöffnungen am Rand des WC-Beckens 34 endet. Das Spühlrohr 42 kann zweiteilig sein und ein senkrechtes Fallrohr und ein waagerechtes Verbindungsstück 44 aufweisen. Die Entleerung des WC-Beckens 34 erfolgt über ein Abflussrohr 48, das an einem Abflussstutzen 50 des WC-Beckens 34 angesetzt ist. Ein Abflussverbindungsstück 46 kann dazwischen gesetzt sein. Der Spülkasten 40 weist die üblichen Bedienungsarmaturen auf, die in den Figuren jedoch nicht dargestellt sind. In allen Anwendungsbeispielen kommt dabei dasselbe DOM 10 zum Einsatz, wobei jeweils nur das Ansaugrohr 22 den unterschiedlichen Verhältnissen angepasst wird. Das Gehäuse 12 des DOM 10 ist insgesamt ein im Wesentlichen rechtwinkliger Körper mit einer maximalen Seitenlänge von etwa 100 mm.

Bei der in Fig. 3 und 4 gezeigten Anwendung wird die Luft über einen im Sitzbrett 36 verlaufenden Kanal 52 angesaugt. Die Einlassöffnung des Kanals 52 befindet sich am hinteren Ende der Öffnung des Sitzbrettes 36. Der Kanal 52 kann sich auch in eine Vielzahl von kleineren Kanälen auffächern, deren Einlassöffnungen über den Rand der Öffnung des Sitzbrettes 36 verteilt sind. Der Kanal 52 mündet über ein Drehrohrgelenk in das Ansaugrohr 22, das zum DOM 10 führt, das in einem Hohlraum im hinteren Bereich des WC-Beckens 34 angeordnet ist. Handelsübliche WC-Becken haben im hinteren Bereich über oder unter dem Abflussstutzen 50 einen geeigneten Hohlraum für die Unterbringung des DOM 10. Der Anschluss des WC-Beckens 34 an den Spülkasten 40 und das Abflussrohr 48 erfolgt mittels üblicher Verbindungsteile und Manschetten.

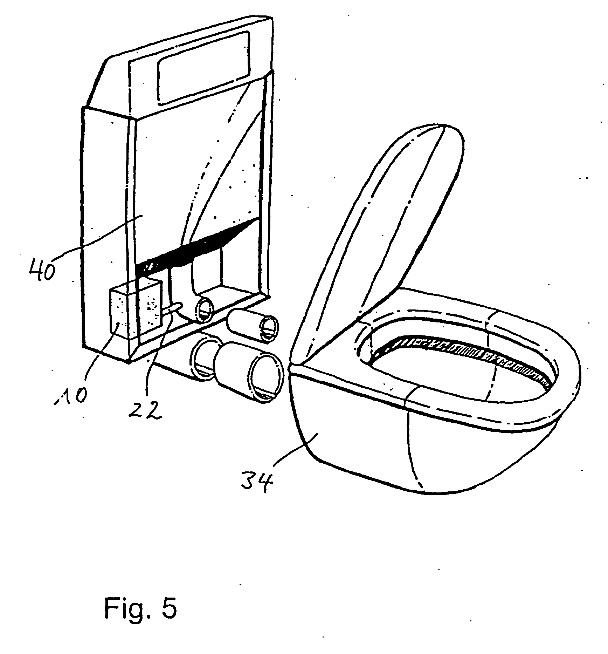

Bei der in Fig. 5 dargestellten Anwendung ist das Ansaugrohr 22 an der Stelle des Spülrohrs 42 angesetzt, an der dieses aus der Waagerechten nach oben abbiegt. Das Ansaugrohr 22 kann dabei stumpf mit dem Spülrohr 42 verbunden sein oder kann sich innerhalb des Spülrohrs 42 ganz oder teilweise bis zum WC-Becken 34 erstrecken. Das DOM 10 befindet sich unten im Spülkasten 40.

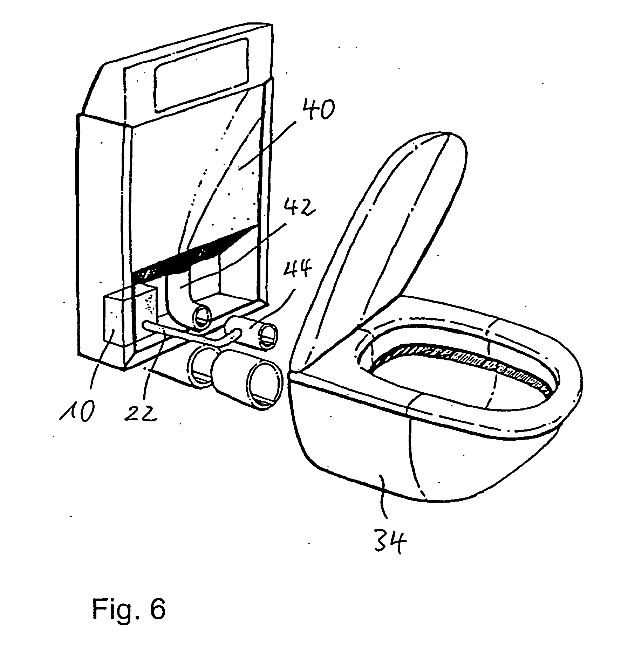

Bei der in Fig. 6 gezeigten Anwendung ist der Anschluss des Ansaugrohrs 22 in Richtung WC-Becken 34 versetzt, und zwar mündet das Ansaugrohr 22 in das Verbindungsstück 44 des Spülrohrs 42 zum WC-Becken 34.

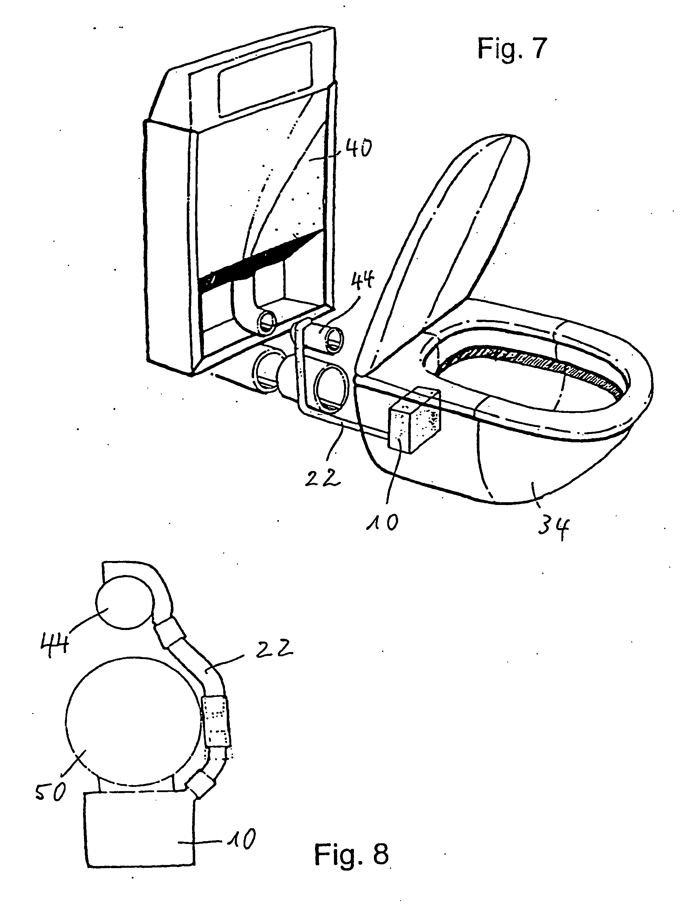

Bei der in Fig. 7 und 8 gezeigten Anwendung ist das Ansaugrohr 22 ebenfalls an das Verbindungsstück 44 angesetzt. Das DOM 10 wird mittels einer Manschette am Abflussstutzen 50 des WC-Beckens 34 abgehängt. Das Ansaugrohr 22 führt von der Oberseite des Verbindungsstücks 44 am Abflussstutzen 50 vorbei und mündet schräg von oben in das DOM 10 (Fig. 8). Ein Vorteil dieser Variante besteht darin, dass am Spülkasten 40 und der Keramik des WC-Beckens 34 keinerlei Änderungen erforderlich sind.

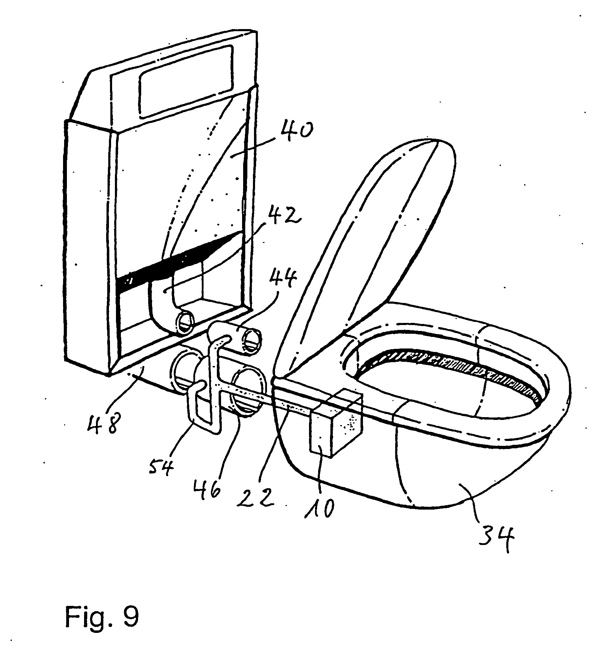

Bei der in Fig. 9 gezeigten Anwendung verbindet ein Verbindungsrohr 54 das Verbindungsstück 44 des Spülrohrs 42 mit dem Abflussrohr 48 oder dem Abflussverbindungsstück 46. Das Verbindungsrohr 54 ist dabei zunächst nach unten und dann wieder wird. Von diesem Verbindungsrohr 54 zweigt oberhalb des Niveaus des Abflussrohrs 48 das Ansaugrohr 22 ab und führt zum DOM 10. Durch das Verbindungsrohr 54 wird Wasser, das aus dem Spülrohr 42 oder dem Verbindungsstück 44 in das Verbindungsrohr 54 austritt, direkt in das Abflussrohr 48 abgeleitet, so dass es nicht den DOM 10 erreichen kann.

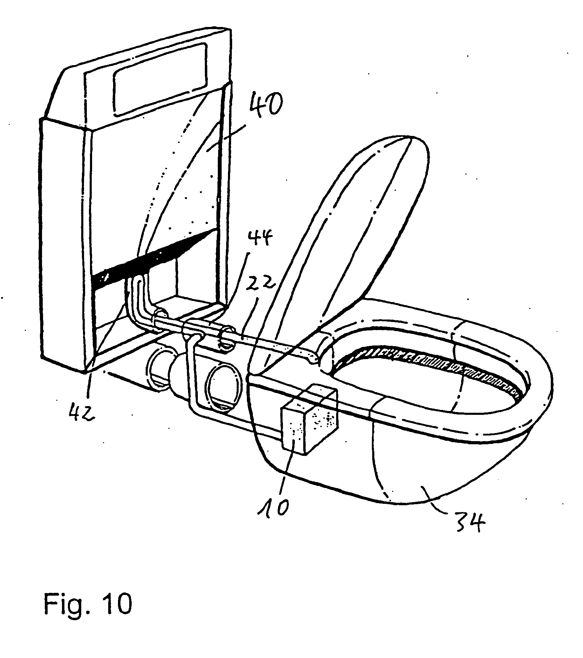

Eine andere Möglichkeit, das DOM 10 vor Wasser zu schützen, ist in Fig. 10 gezeigt. Das Einlassende des Ansaugrohrs 22 befindet sich am Rand des WC-Beckens 34. Das Ansaugrohr 22 verläuft von dort innerhalb des waagerechten Verbindungsstücks 44 und von dort ein Stück in den senkrechten Abschnitt des Spülrohrs 42 , kehrt dann nach unten um, tritt aus dem Verbindungsstück 44 seitlich aus und erreicht schließlich das DOM 10, das hierbei wiederum im hinteren Bereich des WC-Beckens 34 eingebaut ist. Der höchste Punkt des Ansaugrohrs 22, das ist die Umkehrschleife im senkrechten Abschnitt des Spülrohrs 42, liegt höher als der Rand des WC-Beckens 34, damit auch bei einem Überlauf des WC-Beckens 34 kein Wasser über das Ansaugrohr 22 zu dem DOM 10 fließt.

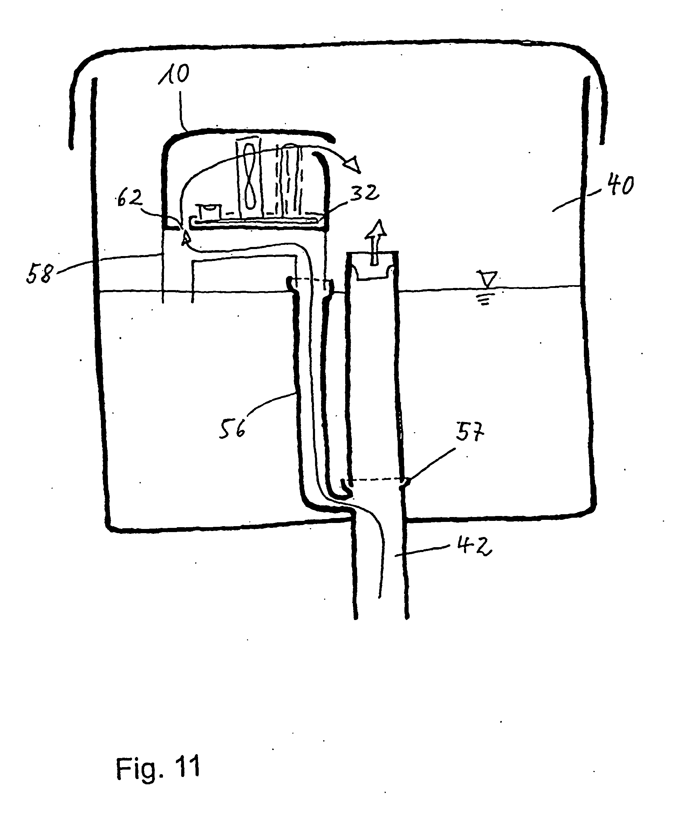

Bei der Anwendung, die in Fig. 11 gezeigt ist, ist das DOM 10 im oberen Bereich des Spülkastens 40 eingebaut. Bei dieser Bauart des Spülkastens ist ein Überlaufrohr 56 vorgesehen, das unterhalb des Spülventils 57 von dem Spülrohr 42 abzweigt und nach oben geführt ist. Zur Anordnung des DOM 10 ist auf das obere Ende des Überlaufrohrs 56 ein Adapter 58 aufgesetzt, in dem ein U-förmiger Kanal verläuft, so dass das obere Ende des Überlaufrohrs 56 nach unten umgeleitet wird. Durch den Adapter 58 behält das Überlaufrohr 56 seine Funktion bei. Bei einem drohenden Überlauf steigt das Wasser zunächst in dem freien Schenkel des U-förmigen Kanals hoch und fließt dann über das Überlaufrohr 56 ab. Der freie Schenkel ist soweit nach unten geführt, dass seine Öffnung bei normaler Füllhöhe des Spülkastens 40 in das Wasser eintaucht. Auf der Oberseite des Adapters 58 ist eine Öffnung 62 vorgesehen und das DOM 10 wird so auf den Adapter 58 aufgesetzt, dass durch die Öffnung 62 die Luft angesaugt wird. Aus dem Inneren des WC-Beckens 34 wird die Luft somit über das Spülrohr 42, das Überlaufrohr 56 und den damit verbundenen Schenkel des U-förmigen Kanals des Adapters 58 und durch die Öffnung 62 hindurch angesaugt. Während der Toilettenspülung kann keine Luft aus dem Inneren des WC-Beckens 34 angesaugt werden. Wenn überschüssiges Wasser über den Adapter 58, das Überlaufrohr 56 und das Spülrohr 42 abfließt, hängt es von der Menge des abfließenden Wassers ab, ob noch ein freier Querschnitt innerhalb dieser Rohre für das in Gegenrichtung erfolgende Absaugen von Luft aus dem Inneren des WC-Beckens 34 vorhanden ist. Das DOM 10 wird bei dieser Ausführungsform im Allgemeinen so auf den Adapter 58 aufgesetzt, dass sich die Platine 32 im unteren Bereich des Gehäuses 10 befindet.

Sobald der Wasserspiegel beim Spülen soweit abgesunken ist, dass der freie Schenkel des U-förmigen Kanals des Adapters 58 nicht mehr ins Wasser eintaucht, so wird durch diesen Schenkel Luft angesaugt, die dann jedoch nicht aus dem Inneren des WC-Beckens 34 kommt.

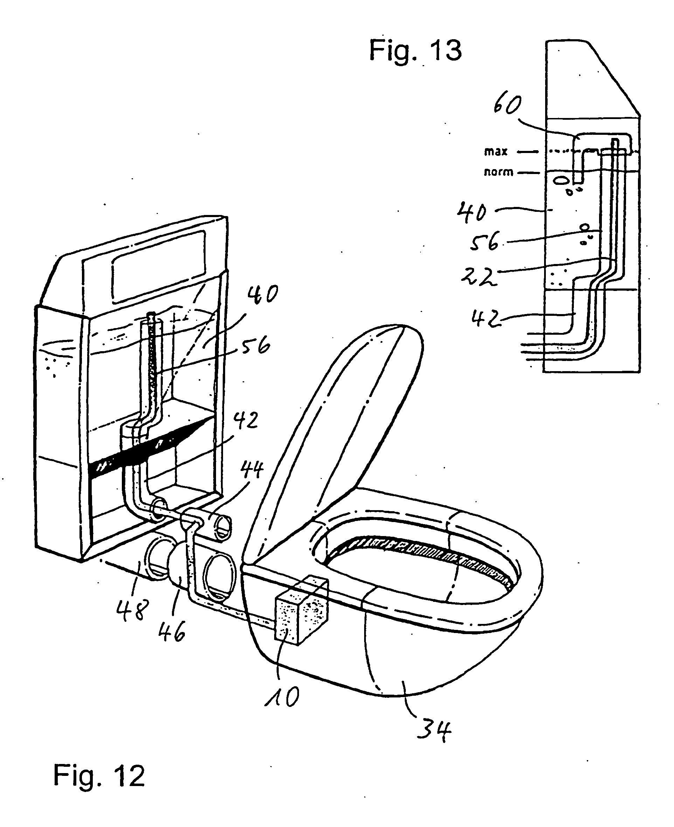

Eine weitere Anwendung ist in den Figuren 12 und 13 gezeigt. Der Spülkasten 40 mit dem Spülrohr 42 und dem Überlaufrohr 56 ist so wie bei der vierten Ausgestaltung der Erfindung ausgebildet. Das Ansaugrohr 22 verläuft jedoch innerhalb des Überlaufrohrs 56, und dann weiter im Spülrohr 42, tritt aus dessen Verbindungsstück 44 seitlich aus und führt zum DOM 10, das wiederum mittels einer Manschette vom Abflussrohr 48 abgehängt ist. Das Einlassende des Ansaugrohrs 22 steht nach oben aus dem Überlaufrohr 56 vor. Um zu verhindern, dass das Ansaugrohr 22 Luft aus dem Spülkasten 40 ansaugt, ist auf das Überlaufrohr 56 ein U-Stück 60 aufgesetzt, das ähnlich dem Adapter 58 der vierten Ausgestaltung der Erfindung ausgebildet ist, jedoch keine Öffnung 62 an der Oberseite aufweist. Bei normaler Füllung des Spülkastens 40 ist das obere Ende des Überlaufrohrs 56 für Lufteintritt aus dem Spülkasten 40 verschlossen, so dass das Ansaugrohr 22 bei vollem Spülkasten Luft nur über das Überlaufrohr 56 und das Spülrohr 42 aus dem Inneren des WC-Beckens 34 ansaugen kann.

## Patentansprüche

1. Vorrichtung zum Vermeiden von lästigen Gerüchen auf Toiletten, mit einem Lüfter (18) zum Absaugen von Luft aus dem Innenbereich des WC-Beckens (34), mit einer Ionisationseinrichtung (24) zum Erzeugen von Luftionen, wobei die abgesaugte Luft mit den erzeugten Luftionen in Kontakt gebracht wird, und mit einem Sensor (16), der die abgesaugte Luft auf oxidierbare Geruchsmoleküle untersucht und ein Signal erzeugt, das die Konzentration oxidierbarer Geruchsmoleküle in der abgesaugten Luft wiedergibt, wobei die Ionisationseinrichtung (24) in Abhängigkeit von dem Signal des Sensors (16) gesteuert wird, und der Sensor ferner auch den Lüfter steuert, **dadurch gekennzeichnet, dass** die Steuerung des Lüfters (18) in der Weise erfolgt, dass der Lüfter mit einer ersten, relativ niedrigen Leistung arbeitet, wenn das Signal des Sensors (16) keine oxidierbaren Geruchsmoleküle in der abgesaugten Luft anzeigt, und dass er mit einer zweiten, höheren Leistung arbeitet, sobald das Signal das Vorhandensein oxidierbarer Geruchsmoleküle in der abgesaugten Luft anzeigt und dass die Steuerung der Ionisationseinrichtung (24) beinhaltet, dass die Ionisationseinrichtung (24) aktiviert wird, sobald der Sensor (16) anzeigt, dass in der abgesaugten Luft oxidierbare Geruchsmoleküle enthalten sind.

2. Vorrichtung nach Anspruch 1, wobei die Ionisationseinrichtung nach dem Prinzip der dielektrisch behinderten Entladung arbeitet und mit Hochspannungs-Rechteckimpulsen hoher Frequenz und konstanter Amplitude betrieben wird und die Steuerung der Ionisationsleistung durch Ändern des Tastverhältnisses erfolgt.

3. Vorrichtung nach Anspruch 1 oder 2, wobei der Sensor ein Wasserstoff-Sensor ist.

4. Vorrichtung nach einem der Ansprüche 1 bis 3, wobei ein Aktivkohlefilter vorgesehen ist, durch das der Luftstrom nach dem Durchgang durch die Ionisationseinrichtung (24) hindurchgeleitet wird.

5. Verfahren zum Vermeiden von lästigen Gerüchen auf Toiletten, wobei mittels eines Lüfters (18) Luft aus dem Innenbereich des WC-Beckens (34) abgesaugt wird, mittels einer Ionisationseinrichtung (24) Luftionen erzeugt werden, die abgesaugte Luft mit den erzeugten Luftionen in Kontakt gebracht wird, mittels eines Sensors (16) die abgesaugte Luft auf oxidierbare Geruchsmoleküle untersucht wird und ein Signal erzeugt wird, das die Konzentration oxidierbarer Geruchsmoleküle in der abgesaugten Luft wiedergibt, und die Ionisationseinrichtung (34) in Abhängigkeit von dein Signal des Sensors (16) gesteuert wird, und der Sensor (16) ferner auch den Lüfter (18) steuert, **dadurch gekennzeichnet, dass** die Steuerung des Lüfters (18) in der Weise erfolgt, dass er mit einer ersten, relativ niedrigen Leistung arbeitet, wenn das Signal des Sensors (16) keine oxidierbaren Geruchsmoleküle in der abgesaugten Luft anzeigt, und dass er mit einer zweiten, höheren Leistung arbeitet, sobald das Signal das Vorhandensein oxidierbarer Geruchsmoleküle in der abgesaugten Luft anzeigt, und dass die Steuerung der Ionisationseinrichtung (24) beinhaltet, dass die Ionisationseinrichtung (24) aktiviert wird, sobald der Sensor (16) anzeigt, dass in der abgesaugten Luft oxidierbare Geruchsmoleküle enthalten sind.

## Claims

1. Device for preventing disagreeable odours on toilets, with an extractor (18) for sucking out air from the inside of the toilet bowl (34), with an ionizer (24) for generating air ions, wherein the extracted air is brought into contact with the generated air ions, and with a sensor (16) which examines the extracted air for oxidizable odour molecules and generates a signal which reproduces the concentration of oxidizable odour molecules in the extracted air, wherein the ionizer (24) is controlled according to the signal from the sensor (16), and the sensor moreover also controls the extractor, **characterized in that** the control of the extractor (18) takes place in such a way that the extractor operates at a first, relatively low capacity if the signal from the sensor (16) does not show oxidizable odour molecules in the extracted air and operates with a second, higher capacity if the signal shows the presence of oxidizable odour molecules in the extracted air and **in that** the control of the ionizer (24) includes the activation of the ionizer (24) as soon as the sensor (16) shows that the extracted air contains oxidizable odour molecules.

2. Device according to claim 1, wherein the ionizer operates according to the principle of dielectrically impeded discharge and operates with high-voltage square pulses of high frequency and constant amplitude and the control of the ionization capacity takes place by changing the pulse duty ratio.

3. Device according to one of claims 1 or 2, wherein the sensor is a hydrogen sensor.

4. Device according to one of claims 1 to 3, wherein an activated-carbon filter is provided through which the air flow is conducted after passing through the ionizer (24).

5. Method for preventing disagreeable odours on toilets, wherein air is extracted of the inside of the toilet bowl (34) by means of an extractor (18), air ions are generated by means of an ionizer (24), the extracted air is brought into contact with the generated air ions, the extracted air is examined for oxidizable odour molecules by means of a sensor (16) and a signal is generated which reproduces the concentration of oxidizable odour molecules in the extracted air, and the ionizer (34) is controlled according to the signal from the sensor (16), and the sensor (16) moreover also controls the extractor (18), **characterized in that** the control of the extractor (18) takes place in such a way that it operates at a first, relatively low capacity if the signal from the sensor (16) does not show oxidizable odour molecules in the extracted air and operates at a second, higher capacity if the signal shows the presence of oxidizable odour molecules in the extracted air and **in that** the control of the ionizer (24) includes the activation of the ionizer (24) as soon as the sensor (16) shows that the extracted air contains oxidizable odour molecules.

## Revendications

1. Dispositif visant à éviter des odeurs nauséabondes sur les toilettes, avec un ventilateur (18) en vue de l'aspiration d'air hors du domaine interne de la cuvette de WC (34), avec un équipement d'ionisation (24) en vue de la production d'ions d'air, l'air aspiré étant mis en contact avec les ions d'air produits, et avec un détecteur (16), qui examine l'air aspiré pour ce qui est de molécules odoriférantes oxydables et qui produit un signal, qui représente la concentration des molécules odoriférantes oxydables dans l'air aspiré, l'équipement d'ionisation (24) étant commandé en fonction du signal provenant du détecteur (16) et le détecteur commandant en outre aussi le ventilateur, **caractérisé en ce que** la commande du ventilateur (18) se fait de telle manière que le ventilateur travaille à une première puissance relativement basse lorsque le signal du détecteur (16) n'indique aucune molécule odoriférante oxydable dans l'air aspiré et qu'il travaille à une deuxième puissance plus élevée lorsque le signal indique la présence de molécules odoriférantes oxydables dans l'air aspiré et **en ce que** la commande de l'équipement d'ionisation (24) contient le fait que l'équipement d'ionisation (24) est activé dès que le détecteur (16) indique que, dans l'air aspiré, sont contenues des molécules odoriférantes oxydables.

2. Dispositif selon la revendication 1, l'équipement d'ionisation travaillant selon le principe de la décharge rendue incomplète par voie diélectrique et étant opéré avec une impulsion de tension rectangulaire à haute fréquence et à amplitude constante et la commande de la puissance d'ionisation se faisant par modification du rapport cyclique de tension.

3. Dispositif selon l'une quelconque des revendications 1 ou 2, le détecteur étant un détecteur à l'hydrogène.

4. Dispositif selon l'une quelconque des revendications 1 à 3, un filtre à charbon actif étant prévu, à travers lequel le courant d'air est acheminé après le passage à travers l'équipement d'ionisation (24).

5. Procédé visant à éviter des odeurs nauséabondes sur les toilettes, de l'air étant aspiré à l'aide d'un ventilateur (18) hors du domaine interne de la cuvette de WC (34), des ions d'air étant produits à l'aide d'un équipement d'ionisation (24), l'air aspiré étant mis en contact avec les ions d'air produits, l'air aspiré étant examiné pour ce qui est de molécules odoriférantes oxydables à l'aide d'un détecteur (16) et un signal étant produit, lequel signal représente la concentration des molécules odoriférantes oxydables dans l'air aspiré, et l'équipement d'ionisation (24) étant commandé en fonction du signal provenant du détecteur (16) et le détecteur (16) commandant en outre aussi le ventilateur (18), **caractérisé en ce que** la commande du ventilateur (18) se fait de telle manière que le ventilateur travaille à une première puissance relativement basse lorsque le signal du détecteur (16) n'indique aucune molécule odoriférante oxydable dans l'air aspiré et qu'il travaille à une deuxième puissance plus élevée lorsque le signal indique la présence de molécules odoriférantes oxydables dans l'air aspiré et **en ce que** la commande de l'équipement d'ionisation (24) contient le fait que l'équipement d'ionisation (24) est activé dès que le détecteur (16) indique que, dans l'air aspiré, sont contenues des molécules odoriférantes oxydables.
